Europäisches Patentamt

European Patent Office

Office européen des brevets

(19)

(11) Veröffentlichungsnummer : **0 173 240**
**B1**

(12)

# EUROPÄISCHE PATENTSCHRIFT

(45) Veröffentlichungstag der Patentschrift :
24.01.90

(21) Anmeldenummer : 85110480.2

(22) Anmeldetag : 21.08.85

(51) Int. Cl.⁵ : **A 61 K 31/52**, A 61 K 9/08,
A 61 K 47/00

(54) Injektionslösung.

(30) Priorität : 29.08.84 DE 3432112

(43) Veröffentlichungstag der Anmeldung :
05.03.86 Patentblatt 86/10

(45) Bekanntmachung des Hinweises auf die Patenterteilung : 24.01.90 Patentblatt 90/04

(84) Benannte Vertragsstaaten :
AT BE CH DE FR GB IT LI LU NL SE

(56) Entgegenhaltungen :
DE--C-- 889 747
GB--A-- 823 242

(73) Patentinhaber : ASCHE AKTIENGESELLSCHAFT
Fischersallee 49-59 Postfach 50 01 32
D-2000 Hamburg 50 (DE)

(72) Erfinder : Loebel, Klaus-Dieter, Dr.
Heinrich-Heine Weg 41
D-2050 Hamburg 80 (DE)

EP 0 173 240 B1

## Beschreibung

Die vorliegende Erfindung betrifft den in dem Patentanspruch gekennzeichneten Gegenstand.

Das 2-Hydroxy-N,N,N-trimethylethanaminium-Salz des 3,7-Dihydro-1,3-dimethyl-1H-purin-2,6-dions (1:1) (Cholintheophyllinat) ist bekanntlich eine pharmakologisch wirksame Substanz, welche als Wirkstoff in broncholytisch wirksamen Arzneimitteln enthalten ist (« Rote Liste 1980 » — Editio Cantor Verlag, Aulendorf, Württemberg DT, Nr. 27 042). Dieses Salz des Theophyllins hat gegenüber anderen Salzen dieser Substanz, wie zum Beispiel dem Theophyllin-Ethylendiamin (= Aminophyllin) den Vorzug, daß es besser verträglich ist. (Arzneim. Forsch., 31, 1981, 1503 ff und 32, 1982, 409 ff). Die das Cholintheophyllinat enthaltenden Präparate werden oral appliziert. Dies hat zur Folge, daß die Konzentration des Wirkstoffs im Blutplasma erst nach etwa zwei Stunden ihr Maximum erreicht (Brit. J. Pharmacol., 3, 1976, Seiten 194 bis 196), was insbesondere bei der Behandlung schwerer Krampfzustände von Nachteil ist. Es besteht somit ein Bedarf an Injektionsmitteln, die diesen Wirkstoff enthalten.

Wässrige Lösungen von Cholintheopyllinat sind nicht stabil und somit als Injektionsmittel ungeeignet. Versucht man, diese Lösungen durch Zugabe von in der Galenik üblicherweise verwendeten Säuren oder Antioxidantien (Ameisensäure, Essigsäure, Phosphorsäure, Benzoesäure, Glutarsäure, Adipinsäure, Sorbinsäure, Apfelsäure, Weinsäure, Ascorbinsäure, Citronensäure etc.) zu stabilisieren, fällt das Cholintheophyllinat aus, oder man erhält ebenfalls instabile sich verfärbende und nicht sterilisierbare Lösungen.

Es wurde nun gefunden, daß man überraschenderweise stabile wässrige Lösungen von Cholintheophyllinat erhält, wenn man dieser Lösung bezogen auf das Salz 5 bis 8, vorzugsweise 6 bis 7 und insbesondere 6,5 bis 6,8 Gewichtsprozent Propionsäure zusetzt. Die erhaltenen Lösungen haben einen $p_H$-Wert von etwa 9 bis 10 — vorzugsweise 9,4 bis 9,8. Sie können in üblicher Weise in Ampullen oder dergleichen abgefüllt und sterilisiert werden.

In diesen wässrigen Injektionslösungen beträgt die Konzentration an Cholintheophyllinat 1 bis 25, vorzugsweise 2 bis 20 und insbesondere 3 bis 15, Gewichtsprozent.

Die erfindungsgemäßen wässrigen Injektionslösungen werden unter anderem zur Behandlung akuter und schwerer Krampfzustände der Bronchialwege bei chronischer Bronchitis, Lungenemphysem und Bronchialasthma — insbesondere bei Status asthmaticus — angewendet. Die applizierte Initialdosis sollte vorzugsweise 5 bis 10 mg/kg und insbesondere 7 bis 9 mg/kg Körpergewicht betragen, die applizierte Tagesdosis sollte etwa in den Grenzen von 15 bis 40 mg/kg und insbesondere in den Grenzen von 19 bis 31 mg/kg Körpergewicht liegen.

Die nachfolgenden Beispiele dienen zur Erläuterung der erfindungsgemäßen wässrigen Injektionslösung.

Beispiel 1

Man bereitet aus
300,00 g Cholintheophyllinat
20,00 g Propionsäure und
9730,00 g bidestilliertem Wasser
10,00 Liter einer Injektionslösung, die sterilfiltriert und unter sterilen Bedingungen in Ampullen zu 10,00 ml abgefüllt werden.

Beispiel 2

Man bereitet aus
150,00 g Cholintheophyllinat
10.00 g Propionsäure und
875,00 g bidestilliertem Wasser
1,00 Liter einer Injektionslösung, die sterilfiltriert und in Ampullen zu 1,00 ml abgefüllt wird.

## Patentanspruch

Wässrige Injektionslösung, gekennzeichnet durch einen Gehalt an 1 bis 25 Gewichtsprozent 2-Hydroxy-N,N,N-trimethylethanaminium-Salz des 3,7-Dihydro-1,3-dimethyl-1H-purin-2,6-dions (1:1) und bezogen auf dieses Salz 5 bis 8 Gewichtsprozent Propionsäure.

## Claim

Aqueous injection solution, characterised by a content of from 1 to 25 % by weight of the 2-hydroxy-N,N,N-trimethylethanaminium salt of 3,7-dihydro-1,3-dimethyl-1Hpurine-2,6-dione (1:1) and from 5 to 8 % by weight, based on that salt, of propionic acid.

## Revendication

Solution aqueuse injectable, caractérisée en ce qu'elle contient de 1 à 25 % en poids de sel d'hydroxy-2 N,N,N-triméthyl-éthane-ammonium de la dihydro-3,7 diméthyl-1,3 purine-1H dione-2,6 (1:1) et, par rapport à ce sel, de 5 à 8 % en poids d'acide propionique.